# EUROPEAN PATENT APPLICATION

(11) **EP 2 361 686 A2**
(43) Date of publication of application: **31.08.2011**
(21) Application number: 10174203.9
(22) Date of filing: 26.08.2010
(51) Int. Cl.: B01L 7/00

(54) **Dissolution testing apparatus for pharmaceutical preparations**

(30) Priority: 27.08.2009 JP 2009197225
(71) Applicant: Uniflex Co., Ltd., Tokyo (JP)
(72) Inventor: Hattori, Akira, Chiba-Pref (JP); Kaneko, Youichi, Kanagawa-Pref (JP)
(74) Representative: Epping, Wilhelm

(57) **Abstract**

A dissolution testing apparatus (41) comprises a vessel plate (42) on which a plurality of test vessels (1) are mounted. Each test vessel (1) has a heat generation portion (3), which includes a transparent ring-shaped heat generating sheet member (4A) wrapped around the cylindrical portion (2A) of the vessel (1) so that the heat generation portion (3) is held thereon in a freely detachable manner. Additionally, the heat generating portion (3) includes a transparent pressing sheet member (5). The dissolution testing apparatus (41) has a heat control block (10), which includes a system control unit, a dc power supply and displays. The heat generation portion (3), if defective, can be easily replaced without the need to replace the main body of the vessel (1), thereby providing cost savings. Further, by providing an unobstructed observation window (4C) between an upper side region and a lower side region of the heat generating sheet member (4A), progress of the dissolution of a pharmaceutical preparation inside the container main body (2) can be observed easily from the outside.

## Description

The present invention relates to a dissolution testing apparatus, and the operation thereof, for pharmaceutical preparations, and more particularly, the present invention is directed to a dissolution test vessel that is suitable for use in a dissolution testing apparatus not requiring a constant temperature water bath.

### BACKGROUND OF THE INVENTION

For oral pharmaceutical preparations, a dissolution test method is prescribed by the Japanese Pharmacopoeia, and the dissolution testing apparatuses that can perform this dissolution test method include dissolution test vessels that are heated without using a water bath. In a waterless apparatus, cylindrical heating means are typically provided around dissolution test vessels. It is therefore possible to eliminate the need for maintenance operations such as cleaning the constant temperature water bath and the need for preparatory time heating the water in the bath before the start of the dissolution test.

Moreover, a vibration generation source, such as a motor, that is required to circulate the water in the bath can be removed, and therefore an improvement in dissolution conditions also can be achieved.

In addition, a dissolution test is typically the final test performed on a sample pharmaceutical preparation to observe how the pharmaceutical preparation behaves in the body. It is therefore desirable to be able to easily observe any dissolution variations during testing from the exterior of the dissolution vessel, while controlling the temperature of the dissolution water in the test vessel at a stable test temperature range of 37 ± 0.5 °C, as near to a reference dissolution test temperature of 37 °C, i.e. close to body temperature.

### SUMMARY OF THE INVENTION

The present invention provides a dissolution testing apparatus having a dissolution test vessel that comprises a transparent vessel main body having a cylindrical portion, a dome-shaped bottom portion that is continuous with a lower end of the cylindrical portion, and a ring-shaped collar portion that projects radially outward from an upper end edge of the cylindrical portion, and a heat generation portion further comprising a transparent ring-shaped heat generating sheet member that is wrapped around an outer peripheral surface of the cylindrical portion so as to be held thereon in a freely detachable manner. The ring-shaped heat generating sheet member is formed from a transparent heat generating material and in which an upper side power feeding strip and a lower side power feeding strip are disposed, respectively. An observation window is formed in the intermediate region between the upper side power feeding strip and the lower side power feeding strip.

According to an aspect of the present invention, an embodiment of the dissolution test vessel includes a water temperature detector and a boiling detector. The water temperature and boiling detectors and the upper side and lower side power feeding strips are connected via terminals to a heat control block for the testing vessel.

According to another aspect the present invention, an embodiment of the heat generation portion includes a pressing sheet member that acts to further secure the heat generating sheet member to the dissolution test vessel.

### BRIEF DESCRIPTION OF THE DRAWINGS

Fig. 1 is a perspective view showing an embodiment of a dissolution test vessel according to the present invention;

Fig. 2 is a schematic perspective view illustrating a heat generating portion shown in Fig. 1;

Fig. 3 is a schematic electric connection diagram showing in detail the constitution of a heating control block shown in Fig. 1;

Fig. 4 is a signal waveform diagram illustrating a heating control operation performed on an energization phase control element by a system control unit shown in Fig. 3;

Fig. 5 is a schematic sectional view illustrating the manner in which a water temperature detector is disposed; and

Fig. 6 is a perspective view showing a dissolution testing apparatus employing six of the dissolution test vessels shown in Fig. 1.

### DESCRIPTION OF THE PREFERRED EMBODIMENTS

An embodiment of the present invention will be described in detail below with reference to the drawings.

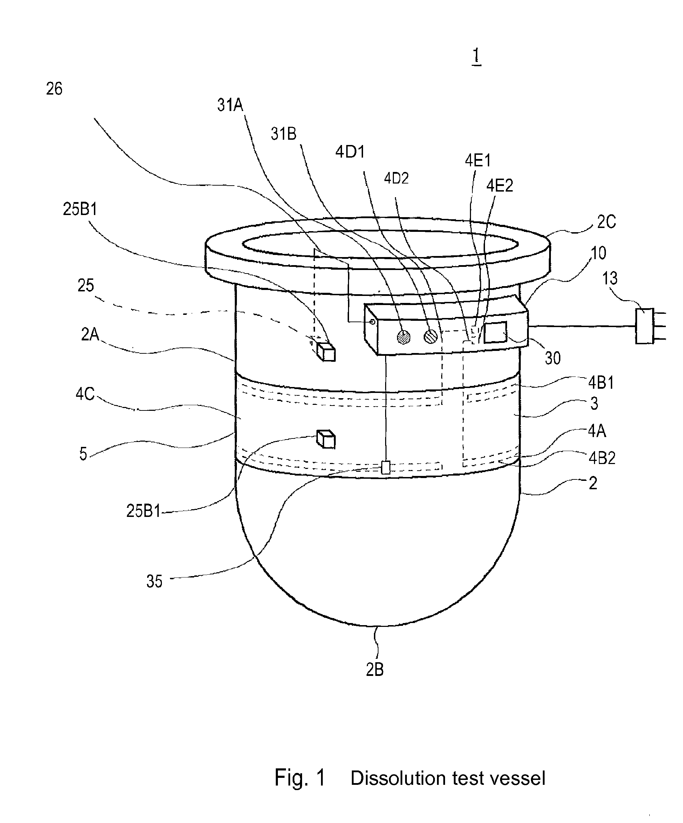

In Fig. 1, a reference numeral 1 denotes an overall dissolution test vessel in which a vessel main body 2 formed from glass or a transparent, chemically inactive material, as prescribed by the Japanese Pharmacopoeia, includes a cylindrical portion 2A, a hemispherical dome-shaped bottom portion 2B that closes a lower end surface of the cylindrical portion 2A, and a ring-shaped collar portion 2C that projects radially outward from a peripheral edge of an upper end surface of the cylindrical portion 2A.

A strip-form heat generation portion 3 is wrapped around the entire periphery of an outer peripheral surface of the cylindrical portion 2A of the vessel main body 2.

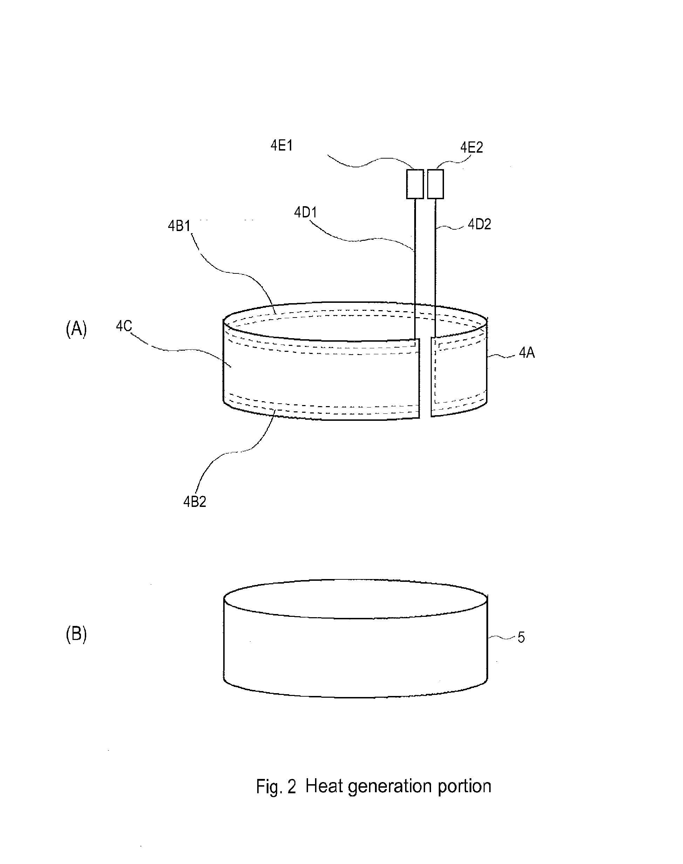

As shown in Fig. 2A, the heat generation portion 3 is transparent, and includes a heat generating sheet member 4A constituted by a rectangular sheet-form transparent heat generating synthetic resin material formed into a ring shape, and an upper side power feeding strip 4B1 and a lower side power feeding strip 4B2 embedded in an upper end edge portion region and a lower end edge portion region of the heat generating sheet member 4A, respectively. Thus, a heat generating current flows through an intermediate region, i.e. the part of the transparent sheet material between the upper side power feeding strip 4B1 and the lower side power feeding strip 4B2 of the heat generating sheet member 4A, and as a result, surface heat is generated in the intermediate region.

The upper side power feeding strip 4B1 and the lower side power feeding strip 4B2 are respectively connected to power feeding terminals 4E1 and 4E2, which are provided in upper portion positions on the outer peripheral surface of the vessel main body 2, via respective power feeding lines 4D1 and 4D2.

The upper side power feeding strip 4B1 is led to an upper end portion on one end edge of the heat generating sheet member 4A and thereby connected to a cool side power feeding line 4D1, while the lower side power feeding strip 4B2 is led from a lower side portion of the heat generating sheet member 4A to the upper end portion along the other end edge and thereby connected to a hot side power feeding line 4D2.

Accordingly, a heating current supplied between the power feeding lines 4D1 and 4D2 travels along the upper side power feeding strip 4B1 and the lower side power feeding strip 4B2 provided on the upper side edge and lower side edge of the power generating sheet member 4A, respectively, whereby the heating current is distributed to various length direction parts of the ring-shaped heat generating sheet member 4A. The current is dispersed to the intermediate surface region between the upper side power feeding strip 4B1 and the lower side power feeding strip 4B2, and as a result, heat is generated over the entire intermediate surface region.

Hence, the heat generating sheet member 4A can be heated substantially evenly as a surface heat generation source for heating the outer peripheral surface of the cylindrical portion 2A of the vessel main body 2, and since the intermediate region part that is subjected to surface heating is transparent, the intermediate region part forms an observation window 4C through which dissolution variation in a pharmaceutical preparation in the interior of the vessel main body 2 can be observed from the exterior of the heat generating sheet member 4A.

In this embodiment, as shown in Fig. 2B, a cylindrical, transparent pressing sheet member 5 formed from a heat-shrinking synthetic resin material is provided around an outer side of the heat generating sheet member 4A, which is disposed on the outer surface of the cylindrical portion 2A of the vessel main body 2, so as to cover the heat generating sheet member 4A.

In a state where the heat generating sheet member 4A is wrapped around the cylindrical portion 2A of the vessel main body 2, the pressing sheet member 5 is fitted onto the outside of the heat generating sheet member 4A from the bottom portion 2B side of the vessel main body 2 so as to overlap the heat generating sheet member 4A.

In this state, heat treatment is applied to the pressing sheet member 5 from the outside, causing the pressing sheet member 5 to shrink such that a shrinkage force is generated in a direction corresponding to a circumferential direction. As a result, an inward radial pressing force is applied to the entire heat generating sheet member 4A.

Thus, the entire heat generating sheet member 4A is pressed and held by the pressing sheet member 5 so as to be wrapped around the outer surface of the cylindrical portion 2A of the vessel main body 2.

As the pressing sheet member 5, a "Teflon (registered trademark) PFA Heat Shrink Tube" PKF-200-110B, manufactured by Packing Land Ltd., may be used.

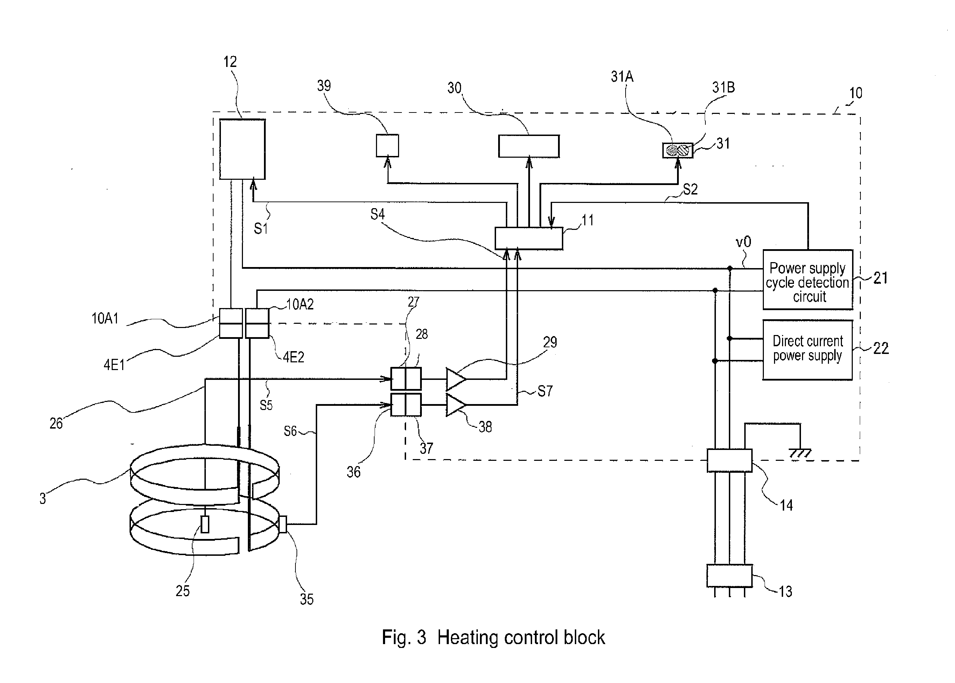

As shown in Fig. 3, the power feeding terminals 4E1 and 4E2 are connected to connection terminals 10A1 and 10A2 that are provided on an inside surface of a heating control block 10, which is fixed externally to an upper portion outer peripheral surface of the cylindrical portion 2A, so as to oppose the cylindrical portion 2A.

The heating control block 10 includes a system control unit 11 constituted by a microcomputer, and by controlling an energization angle of an energization phase control element 12 constituted by a TRIAC in accordance with a phase control signal S1 output by the system control unit 11, an alternating current obtained from a household power supply outlet by a power plug 13 is supplied to the power feeding terminals 4E1 and 4E2 of the heat generation portion 3 via the energization phase control element 12 and the connection terminals 10A1 and 10A2.

In this embodiment, the power plug 13 is constituted by a three-terminal plug including an earth terminal. Hence, a household power supply having an alternating current voltage V0 (100 V in this embodiment) is input into the heating control block 10 via an input terminal 14.

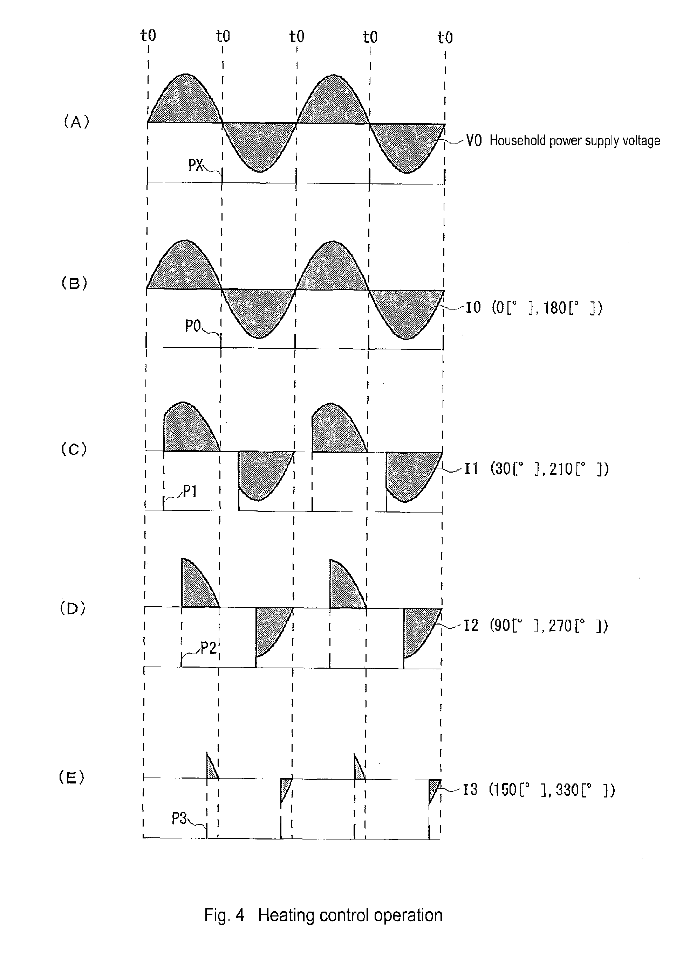

As shown in Fig. 4A, in the household power supply voltage V0, zero-cross is generated cyclically at timings t0 when a power supply phase is 0° and 180°. A power supply cycle detection circuit 21 (Fig. 3) detects this zero-cross and transmits a zero-cross detection pulse PX to the system control unit 11 in the form of a zero-cross detection signal S2.

In this embodiment, when a water temperature detection signal S4 indicates a much lower room temperature than the reference dissolution test temperature of 37 °C, the system control unit 11 trigger-activates the energization phase control element 12 constituted by a TRIAC at the generation timing of the trigger pulse PX. As a result, as shown in Fig. 4B, a heating current 10 is supplied to the heat generation portion 3 in a phase range of 0 to 180° or 180 to 360° with respect to the phase of the household power supply voltage V0.

At this time, the heat generation portion 3 generates maximum thermal energy, and therefore the dissolution water in the vessel main body 2 is heated rapidly.

When, as a result, the temperature of the dissolution water approaches the reference dissolution test temperature of 37 °C, the system control unit 11 retards a trigger phase of a trigger pulse P1, P2 or P3 for activating the energization phase control element 12 on the basis of the water temperature detection signal S4 and in accordance with the increase in the temperature of the dissolution water, as shown in Fig. 4C, 4D or 4E, whereby a heating current I1, 12 or 13, current values of which are progressively smaller, is supplied to the heat generation portion 3.

At this time, the thermal energy generated by the heat generation portion 3 gradually decreases, and therefore the temperature increase rate of the dissolution water in the vessel main body 2 gradually decreases such that the dissolution water eventually reaches the reference dissolution test temperature of 37 °C.

When the temperature of the dissolution water in the vessel main body 2 exceeds the reference dissolution test temperature of 37 °C, on the other hand, the system control unit 11 performs control on the basis of the water temperature detection signal S4 such that a trigger pulse is not applied to the energization phase control element 12, and as a result, a heating current is not supplied to the heat generation portion 3.

Hence, when the temperature of the dissolution water in the vessel main body 2 exceeds the reference dissolution test temperature of 37 °C, the dissolution water radiates heat naturally without being heated, and therefore the temperature falls to or below the reference dissolution test temperature of 37 °C. Accordingly, the system control unit 11 returns to the control state described above in relation to Figs. 4B to 4E.

Hence, the system control unit 11 can control the temperature of the dissolution water in the vessel main body 2 to the dissolution test temperature range of 37 ± 0.5 °C prescribed by the Japanese Pharmacopoeia.

Figs. 4B, 4C, 4D and 4E shows examples in which a heating current 10, I1, 12 or 13 is supplied to the power feeding terminals 4E1 and 4E2 of the heat generation unit 3 when the energization phase control element 12 constituted by a TRIAC is triggered by a power supply voltage V0 having a phase of 0° or 180°, 30° or 210°, 90° or 270°, or 150° or 330°, respectively.

The temperature of the dissolution water in the vessel main body 2 is detected by a water temperature detector 25 disposed on an inside surface of the cylindrical portion 2A of the vessel main body 2, whereupon a water temperature detection output S5 is applied to a temperature detection terminal 28 of the heating control block 10 via a water temperature detection signal line 26 and a detection output terminal 27, in that order.

The water temperature detection output S5 applied to the temperature detection terminals 28 is amplified by a buffer amplifier 29 having a bridge input differential constitution and then supplied to the system control unit 11 as the water temperature detection signal S4.

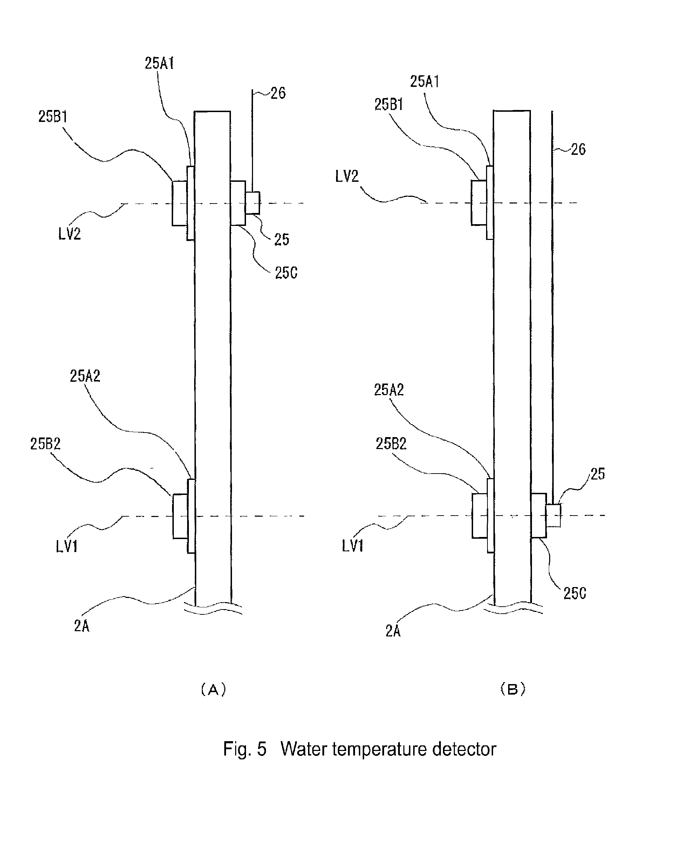

In this embodiment, as shown in Fig. 5A, the water temperature detector 25 is fixed onto an adsorption permanent magnet 25C, which is held on the inside surface of the cylindrical portion 2A by adsorption, by attachment permanent magnets 25B1 and 25B2 that are fixed to adhesive layers 25A1 and 25A2 adhered to the outside surface of the cylindrical portion 2A.

The attachment permanent magnets 25B1 and 25B2 are provided in accordance with the amount of dissolution water injected into the vessel main body 2 such that when the injection amount is 900 ml, the upper water level attachment permanent magnet 25B1 is provided in the position of an upper water level LV2 corresponding to the injection amount, and the lower water level attachment permanent magnet 25B2 is provided in the position of a lower water level LV1 corresponding to a case in which 500 ml of the dissolution water is injected.

Hence, when the dissolution water injected into the vessel main body 2 is at a high water level, a user adsorbs the adsorption permanent magnet 25C to the attachment permanent magnet 25B1 at the upper water level LV2, as shown in Fig. 5A, such that the water temperature detector 25 can detect the temperature of the dissolution water at the high water level.

On the other hand, when the dissolution water injected into the vessel main body 2 is at a low water level, the user adsorbs the adsorption permanent magnet 25C of the water temperature detector 25 to the attachment permanent magnet 25B2 provided at the lower water level LV1, as shown in Fig. 5B, such that the temperature of the dissolution water at the low water level can be detected correctly.

In this embodiment, a liquid crystal temperature display portion 30 is provided on the heating control block 10, and the system control unit 11 displays the temperature of the dissolution water in the vessel main body 2, detected in accordance with the water temperature detection signal S4, thereon so that the user can check the temperature easily.

Further, a heating operation display portion 31 constituted by an LED element is provided on a surface of the heating control block 10, and when the temperature of the dissolution water in the vessel main body 2 is raised from room temperature to the reference dissolution test temperature of 37 °C in preparation for a dissolution test operation, a heating underway display 31A constituted by a red LED is illuminated to notify the user that a heating operation is underway.

When the dissolution water in the vessel main body 2 is in a stable condition within the dissolution test temperature range of 37 ± 0.5 °C, on the other hand, a stable display 31B constituted by a green LED is illuminated to notify the user that a stable heating operation condition has been established.

Further, a boiling detector 35 (Figs. 1 and 3) constituted by a thermistor is provided on the outer peripheral surface of the heat generation portion 3, and a boiling detection output S6 therefrom is input into the system control unit 11 as a boiling detection signal S7 via a detection output terminal 36, a temperature detection terminal 37, and a buffer amplifier 38 having a bridge input differential constitution.

Hence, when the vessel main body 2 reaches an abnormally high temperature, the system control unit 11 detects the abnormally high temperature as boiling and informs the user thereof by generating a warning sound from a boiling alarm 39. The system control unit 11 also interrupts the heat generation operation of the heat generation portion 3 by interrupting output of the phase control signal S1.

In Fig. 3, a reference numeral 22 denotes a direct current power supply for supplying a direct current power supply to each part of the heating control block 10.

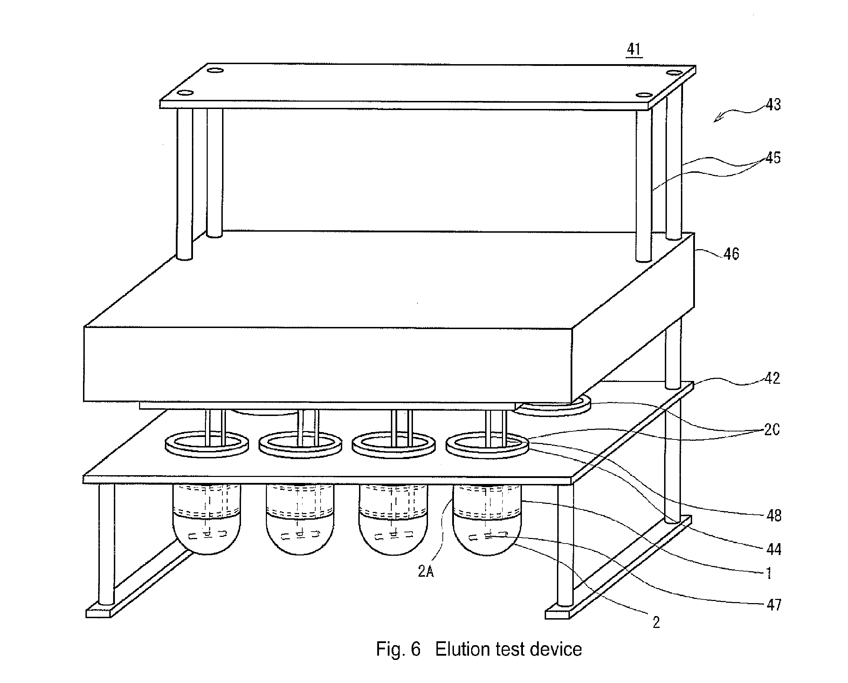

As shown in Fig. 6, the dissolution test vessel 1 constituted as described above is attached to an attachment substrate 42 of a dissolution testing apparatus 41.

In the dissolution testing apparatus 41, six attachment holes 44 are drilled into the attachment substrate 42, which is fixed to a frame 43 so as to extend in a horizontal direction, and the cylindrical portion 2A of the vessel main body 2 is inserted into and held in the six attachment holes 44 from above such that the collar portion 2C contacts the attachment substrate 42. Thus, simultaneous dissolution tests can be performed using the six dissolution test vessels 1 during a single dissolution test operation.

To start a dissolution test in the dissolution testing apparatus 41 using the six dissolution test vessels 1, the user pours test dissolution water into the dissolution test vessels 1 attached to the attachment holes 44 in the attachment substrate 42 after moving a dissolution testing apparatus main body 46 upward along guide rails 45 of the frame 43.

In this embodiment, 900 ml or 500 ml of dissolution water are poured into the vessel main body of the dissolution test vessel 1. The user then lowers the dissolution testing apparatus main body 46 to a predetermined position such that a stirring paddle 47 is inserted into each dissolution test vessel 1 from above, whereupon heating of the heat generation portion 3 is begun.

At this time, the heating control block 10 provided on the vessel main body of each dissolution test vessel 1 starts to heat the dissolution water in the vessel main body 2 rapidly on the basis of a command from the dissolution testing apparatus main body 46 by causing the energization phase control element 12 to pass the heating current 10 (Fig. 4B) having an energization phase of 0° or 180° through the upper side power feeding strip 4B1 and the lower side power feeding strip 4B2. As a result, an energizing current applied to the heat generation portion 3 is phase-controlled on the basis of the water temperature detection signal S4 from the water temperature detector 25 such that the temperature of the dissolution water reaches the reference dissolution test temperature of 37 ± 0.5 °C.

Thus, at the start of the dissolution test, while the temperature of the dissolution water in the respective vessel main bodies 2 of the six dissolution test vessels 1 attached to the dissolution testing apparatus 41 is controlled to the reference dissolution test temperature of 37 ± 0.5 °C and the dissolution water is stirred by the stirring paddles 47, a sample pharmaceutical preparation introduced into the vessel main body 2 from the dissolution testing apparatus main body 46 is steadily eluted into the dissolution water.

As regards the progress of the dissolution condition in each of the dissolution test vessels 1 at this time, since the heat generation portion 3 is formed from a transparent material and the observation window 4C is provided between the upper side power feeding strip 4B1 and the lower side power feeding strip 4B2, the user can observe the progress of the dissolution condition easily from the outside as the pharmaceutical preparation in the vessel main body 2 is dissolved into the dissolution water from a fragmented state.

During observation of the dissolution condition, the dissolution testing apparatus main body 46 extracts dissolution water automatically at predetermined time intervals via a water extraction pipe 48 inserted into each dissolution test vessel 1, and therefore variation in the concentration of the pharmaceutical preparation eluted into the vessel main body 2 can be checked.

When the dissolution test is completed in relation to a single portion of the pharmaceutical preparation, the six dissolution test vessels 1 attached to the dissolution testing apparatus 41 are removed from the attachment substrate 42 and washed, whereupon the dissolution test vessels 1 are reattached to the attachment holes 44 in the attachment substrate 42 for the next dissolution test.

Hence, dissolution tests are performed repeatedly on a large number of pharmaceutical preparations using the same vessel main bodies 2. However, when a defect occurs in relation to the heat generation portion 3 of one of the six dissolution test vessels 1 during this time, the user can perform the dissolution test using the same vessel main bodies 2 by detaching the heat generation portion 3 from the cylindrical portion 2A of the defective dissolution test vessel 1 and wrapping a new heat generation portion 3 around the cylindrical portion 2A.

As shown in Figs. 2A and 2B, the heat generating sheet member 4A bent into a ring shape is wrapped around the cylindrical portion 2A of the vessel main body 2, and in this state, the heat-shrinking pressing sheet member 5 is provided to cover the periphery thereof. Thus, the heat generation portion 3 is pressed against and held on the cylindrical portion 2A so as to be detachable from the outer peripheral surface of the vessel main body 2. Hence, by performing a simple operation of cutting through the defective ring-shaped heat generation portion 3 in a vertical direction, for example, the heat generation portion 3 can be detached easily from the pressed and held state, and thus the heat generation portion 3 can be replaced.

The new heat generation portion 3 can then be wrapped around the outside of the cylindrical portion 2A easily and with stability simply by fitting the new heat generation portion 3 onto the corresponding vessel main body 2 from the bottom portion 2B side and applying heat thereto.

Hence, the user can continue to use the vessel main body 2 to which the defective heat generation portion 3 was attached, and therefore a dramatic improvement in the use efficiency of the vessel main body 2 can be achieved.

Incidentally, to replace the entire vessel main body 2, authorization must be obtained from the Japanese Pharmacopoeia, and therefore, if the heat generation portion 3 cannot be replaced easily, the corresponding vessel main body 2 must be discarded together with the defective heat generation portion 3. With the constitution described above, however, it is possible to replace only the defective heat generation portion 3 without replacing the vessel main body 2, and therefore a dramatic improvement in use efficiency can be achieved.

Note that in the above embodiment, a case in which the dissolution test vessel 1 constituted as shown in Fig. 1 is attached to the dissolution testing apparatus 41 having the six attachment holes 44 shown in Fig. 6 was described. However, the dissolution test vessel 1 is not limited to this application, and similar effects to those described above can be obtained when the dissolution test vessel 1 is provided in a greater number than six, for example twelve, or a smaller number than six, for example one.

Further, in the embodiment described above, as shown in Fig. 2, the heat generation portion 3 is pressed against and held on the cylindrical portion 2A of the vessel main body 2 by providing the pressing sheet member 5 formed from a heat-shrinking synthetic resin material so as to cover the heat generating sheet member 4A and applying heat thereto. However, the heat generation portion 3 is not limited to this constitution, and as long as the heat generation portion 3 provided on the periphery of the cylindrical portion 2A includes a ring-shaped sheet member that can be pressed against and held on the cylindrical portion 2A by a shrinkage force thereof that acts in a direction corresponding to the circumferential direction, any overall constitution may be employed.

Furthermore, in the embodiment described above, the dissolution test vessel 1 is formed on the basis of prescriptions laid down by the Japanese Pharmacopoeia, but the dissolution test vessel 1 may be formed on the basis of prescriptions laid down by another pharmacopoeia, for example the US Pharmacopeia.

This dissolution test vessel may be used in a dissolution test that is performed on a pharmaceutical preparation on the basis of a pharmacopoeia.

## Claims

1. A dissolution testing apparatus comprising:
a vessel plate;
a plurality of transparent test vessels having a cylindrical portion, a dome-shaped bottom portion that is continuous with a lower end of the cylindrical portion, and a collar portion that projects radially outward from an upper end edge of the cylindrical portion;
a heat generation portion having a transparent ring-shaped heat generating sheet member wrapped in a detachable manner around an outer peripheral surface of the cylindrical portion of each transparent test vessel; and
heating control means.

2. The dissolution testing apparatus according to claim 1, wherein the transparent ring-shaped heat generating sheet member comprises a thin transparent heat generating material with an upper-side power feeding strip and a lower-side power feeding strip disposed, respectively, in an upper-side region and a lower-side region of said sheet member, wherein an observation window is formed between said upper-side and said lower-side power feeding strips.

3. The dissolution testing apparatus according to claim 1, wherein the heat generation portion including a detachable transparent ring-shaped pressing sheet member for holding said transparent ring-shaped heat generating sheet member in position against the outer peripheral surface of the cylindrical portion of said test vessel.

4. A dissolution test vessel comprising:
a detachable heat generation portion positioned on a cylindrical portion of said dissolution test vessel, wherein said detachable heat generating portion is capable of being removed and replaced with a new heat generation portion.

5. The dissolution test vessel according to claim 4, wherein the heat generation portion comprises a transparent heat generating sheet member made from a heat generating synthetic resin.

6. The dissolution test vessel according to claim 4, wherein the heat generation portion comprises a transparent heat generating sheet member and a transparent pressing sheet member.

7. The dissolution test vessel according to claim 4, wherein the detachable heat generation portion is pressed against and held in place on the cylindrical portion of said test vessel solely by a shrinkage force that acts in an inward circumferential direction.

8. The dissolution test vessel according to claim 4, wherein the cylindrical portion of said test vessel includes a water temperature detector and a boiling detector.

9. A heating element comprising:
a transparent heat generating synthetic resin sheet having an upper-side power feeding strip and a lower-side power feeding strip wherein an electrical current flows there between said upper-side and lower-side power feeding strips generating heat.

10. The heating element according to claim 9 further comprising a shrinkable, transparent pressing sheet member that holds said transparent heat generating synthetic resin sheet in place.
